# FASCICULE DE BREVET EUROPEEN

(11) **EP 2 315 577 B1**
(45) Date de publication et mention de la délivrance du brevet: **09.11.2016**
(21) Numéro de dépôt: 09740359.6
(22) Date de dépôt: 22.07.2009
(51) Int. Cl.: A61K 8/66, A61K 8/97, A61K 8/36, A61K 8/49, A61K 38/46, A61K 36/00, A61P 17/00, A61Q 19/02, A61Q 19/00, A61K 8/04

(54) **COMPOSITION DERMOCOSMETIQUE, PROCEDE DE TRAITEMENT ESTHETIQUE A PARTIR DE LA COMPOSITION, ET UTILISATION DE LA COMPOSITION POUR ECLAIRCIR LA PIGMENTATION DE LA PEAU**
DERMOKOSMETISCHE ZUSAMMENSETZUNG, ÄSTHETISCHES BEHANDLUNGSVERFAHREN MIT DER ZUSAMMENSETZUNG UND VERWENDUNG DER ZUSAMMENSETZUNG ZUR AUFHELLUNG DER HAUTPIGMENTIERUNG
DERMOCOSMETIC COMPOSITION, AESTHETIC TREATMENT METHOD USING THE COMPOSITION, AND USE OF THE COMPOSITION FOR LIGHTENING THE PIGMENTATION OF THE SKIN

(30) Priorité: 23.07.2008 FR 0855026
(43) Date de publication de la demande: 04.05.2011
(73) Titulaire: ACM, 92110 Clichy (FR)
(72) Inventeur: GAUTHIER Yvon, 33110 Le Bouscat (FR)
(74) Mandataire: Novagraaf Technologies
(86) Numéro de dépôt international: PCT/FR2009/051471
(87) Numéro de publication internationale: WO 2010/010299

(56) Documents cités:
- EP-A1- 0 308 919
- EP-A1- 1 273 283
- EP-A1- 1 586 315
- WO-A1-98/46210
- US-A- 5 840 338
- DATABASE WPI Week 200219 Thomson Scientific, London, GB; AN 2002-143812 XP002624452, -& JP 2001 302440 A (OKUBO M) 31 octobre 2001 (2001-10-31)

## Description

La présente invention concerne, de façon générale, le domaine du traitement de la peau.

Plus précisément, la présente décrit, selon un premier de ses aspects, une composition dermocosmétique visant notamment à éclaircir la pigmentation de la peau.

Une exposition régulière de la peau aux radiations ultraviolettes (UV), ainsi qu'à d'autres stimuli tels qu'inflammation ou frictions mécaniques, favorise la production de mélanine par les mélanocytes. Le mécanisme intime est encore mal connu. Il semble cependant que les UV stimulent l'activité de la tyrosinase, qui permet l'oxydation de précurseurs chimiques et aboutit à la synthèse de mélanine dans les mélanocytes, qui se dépose sur les mélanosomes. Ces mélanosomes une fois mélanisés sont cédés aux kératinocytes voisins des mélanocytes. Ces mélanosomes porteurs de mélanine demeurent un certain temps dans le cytoplasme des kératinocytes.

Au niveau des couches moyennes et supérieures de l'épiderme, chez les caucasoïdes, c'est-à-dire chez les sujets présentant une peau blanche, les mélanosomes mélanisés sont ensuite intégrés dans les lysosomes pour y être progressivement dégradés. Cette dégradation s'effectue progressivement grâce à l'intervention d'un système enzymatique contenu dans les lysosomes, qui inclut notamment les phosphatases acides, les cathepsines, et les arylsulfatases.

Habituellement, dans l'épiderme de sujets caucasoïdes, la mélanine est absente de la couche cornée en raison de sa destruction progressive dans les couches épidermiques superficielles. Il n'en est pas de même dans l'épiderme des sujets présentant une peau noire. Chez ces sujets, les mélanosomes, de taille plus importante, ne sont pas détruits et persistent au niveau de la couche cornée.

Dans certains cas particuliers, on peut observer une surcharge mélanique des cellules épidermiques et parfois des dépôts mélaniques intracellulaires dermiques. C'est le cas du mélasma et des lentigos, qui sont caractérisés par l'apparition de taches hyperpigmentées permanentes et inesthétiques.

Il existe sur le marché de nombreuses préparations qui sont d'une efficacité très variable pour atténuer ces hyperpigmentations qui entrainent un préjudice esthétique certain.

Ces préparations connues ont, pour la plupart, pour objectif de freiner ou d'inhiber la production de mélanine par les mélanocytes en s'attaquant directement à la tyrosinase. C'est le cas de l'hydroquinone et de nombreux produits dont la structure moléculaire est proche de celle de la tyrosine. Certains de ces produits, et par exemple des dérivés de l'hydroquinone, présentent l'inconvénient d'être toxiques pour les mélanocytes.

Les hyperpigmentations peuvent être atténuées aussi grâce à l'utilisation locale d'antioxydants bloquant l'oxydation des précurseurs de la mélanine. Ces antioxydants sont essentiellement l'acide ascorbique, ou des dérivés soufrés.

Dans ce contexte, la présente invention a pour but de proposer une nouvelle utilisation cosmétique d'au moins une phosphatase acide pour éclaircir la peau, l'éclaircissement de la pigmentation de la peau, et notamment à l'éclaircissement des taches hyperpigmentées.

A cette fin, la composition dermocosmétique est décrite et est essentiellement caractérisée en ce qu'elle comprend au moins une hydrolase choisie parmi les phosphatases acides.

Cette composition est bien tolérée par la peau et permet d'obtenir un bon éclaircissement de la peau au bout de quelques semaines.

La composition inclut de préférence entre 0,5 et 15% en poids, tout préférentiellement entre 5 et 10% en poids, d'au moins une hydrolase choisie parmi les phosphatases acides, les cathepsines, et les arylsulfatases.

Les phosphatases acides représentent en fait une famille d'enzymes existant à la fois dans le monde animal et végétal. Dans le monde animal, elles sont secrétées par un certain nombre de cellules telles que cellules de prostate, ou de foie. Chez les végétaux, elles existent en quantité appréciable et leur extraction est plus simple qu'à partir des tissus animaux.

Dans un mode de réalisation préféré de l'invention, les phosphatases acides sont d'origine végétale. Par exemple, elles sont extraites de la pomme de terre, de la patate douce, ou d'une céréale telle que le germe de blé. L'utilisation de phosphatases acides extraites de végétaux présente l'avantage d'être peu coûteuse.

L'utilisation topique de phosphatases acides d'origine végétale semble stimuler la dégradation de la mélanine intra-épidermique ce qui se traduit par un éclaircissement de lésions du mélasma. En outre, 19 utilisation cosmétique de phosphatases acides d'origine végétale selon l'invention est particulièrement bien tolérée par la peau.

Les cathepsines et les arylsulfatases regroupent aussi sous cette appellation plusieurs types d'enzyme.

Les cathepsines peuvent être isolées à partir de tissus animaux (rate de boeuf, placenta humain, ou foie humain). Notamment, la cathepsine D peut être utilisée dans le cadre de l'invention.

Les arylsulfatases, d'origine bactérienne, peuvent être soit extraites à partir des bactéries Aerobacter aerogenes ou Flavobacterium heparinum, ou à partir de tissus animaux (escargot de Bourgogne (Helix pomatia) ou patelle (Patella vulgata)).

L'utilisation de phosphatases acides est choisie de préférence à celle des cathepsines et/ou des arylsulfatases pour des raisons de coût. En effet, à l'heure actuelle, les phosphatases acides sont moins chères à l'achat que les arylsulfatases ou que les cathepsines. L'efficacité de ces différentes familles d'hydrolase est similaire.

La composition dermocosmétique décrite peut comprendre en outre au moins un agent dépigmentant choisi parmi les inhibiteurs de l'hormone mélanotrope ou MSH, les inhibiteurs de l'endothéline-1 ou ET-1, les inhibiteurs de la tyrosinase, les antioxydants, et les agents kératolytiques.

De préférence, une telle composition comprend entre 0,5 et 15% en poids, tout préférentiellement entre 5 et 10% en poids, d'au moins une hydrolase choisie parmi les phosphatases acides, les cathepsines, et les arylsulfatases, et entre 0,5 et 5% en poids d'au moins un agent dépigmentant choisi parmi les inhibiteurs de l'hormone mélanotrope ou MSH, les inhibiteurs de l'endothéline-1 ou ET-1, les inhibiteurs de la tyrosinase, les antioxydants, et les agents kératolytiques.

Notamment, la composition peut comprendre, comme agent dépigmentant, au moins un inhibiteur de la tyrosinase choisi parmi l'hydroquinone et ses dérivés, l'acide kojique, l'arbutine, la glabridine, le rucinol, et l'acide azélaïque, et/ou comprendre au moins un antioxydant choisi parmi l'acide ascorbique et la cystéine et ses dérivés, et/ou comprendre un agent kératolytique choisi parmi l'acide glycolique et l'acide rétinoïque.

L'association dans une même composition dermocosmétique d'un produit stimulant la dégradation de la mélanine, tel que les phosphatases acides, les cathepsines, ou les arylsulfatases, et d'un produit freinant la synthèse de mélanine, tels que des agents dépigmentants connus, offre une efficacité et une tolérance améliorée par rapport aux compositions connues, notamment par rapport à celles qui contiennent uniquement de tels agents dépigmentants connus.

La composition décrite peut se présenter sous l'une des formes galéniques suivantes : crème, pommade, lotion, gel, lait, composition pressurisée pour aérosol, solution susceptible d'être injectée, ou solution susceptible d'être administrée par ionisation.
La composition décrite inclut, en plus des phosphatases acides et éventuellement d'au moins un agent dépigmentant, un ou plusieurs additifs utilisés classiquement dans les compositions dermocosmétiques.

Selon un deuxième aspect, un procédé de traitement esthétique de la peau visant notamment à l'éclaircir, caractérisé en ce que l'on traite la peau, au moins localement, avec une composition décrite.

Selon un mode de réalisation, on traite la peau par application de la composition sur l'épiderme.

Selon un autre mode de réalisation, on traite la peau par injection de la composition à travers l'épiderme.

Selon encore un autre mode de réalisation, on traite la peau par administration de la composition par ionisation.

L'invention concerne, l'utilisation cosmétique d'au moins une phosphatase acide pour éclaircir la peau, et par exemple pour traiter les mélasmas ou masques de grossesse, ou les lentigos (taches de vieillesse ou lentigos solaires).

La composition dermocosmétique décrite peut également être utilisée pour l'éclaircissement des peaux noires.

D'autres caractéristiques et avantages de l'invention ressortiront clairement de la description détaillée qui en est faite ci-après, à titre indicatif et nullement limitatif, en référence aux dessins annexés dans lesquels :
- les figures 1 et 2 représentent chacune une vue en microscopie optique d'un échantillon de peau soit non traité (figure 1, témoin), soit traité avec une composition comprenant des phosphatases acides (figure 2) ;
- les figures 3 et 4 représentent chacune une vue en microscopie électronique de mélanosomes observés dans le cytoplasme de kératinocytes au niveau d'un échantillon d'épiderme soit non traité (figure 3, témoin), soit traité avec une composition comprenant des phosphatases acides (figure 4).

### Exemple 1 : Utilisation de phosphatases acides d'origine végétale pour éclaircir la peau.

### Exemple 1A : Etudes chez l'animal.

Des études expérimentales ont été réalisées sur des cobayes ayant un pelage noir.

Des applications locales biquotidiennes de solutions à 5% en poids de phosphatases acides ont été réalisées sur l'oreille droite des cobayes, l'oreille gauche servant de témoin.

Ces solutions contenaient également de l'eau et un excipient permettant la pénétration de la solution dans l'épiderme, dans l'exemple, le propylène glycol.

On a ainsi observé, dans un délai de 15 jours, un éclaircissement notable visible à l'oeil nu.

En outre, des études histologiques comparatives, en microscopie optique, ont été pratiquées sur des prélèvements (punch de 2 mm de diamètre) sur les oreilles traitées et non traitées.

Des prélèvements cutanés sur des zones de peau non traitées et des zones de peau traitées ont en outre servi à la réalisation d'une étude ultrastructurale en microscopie électronique.

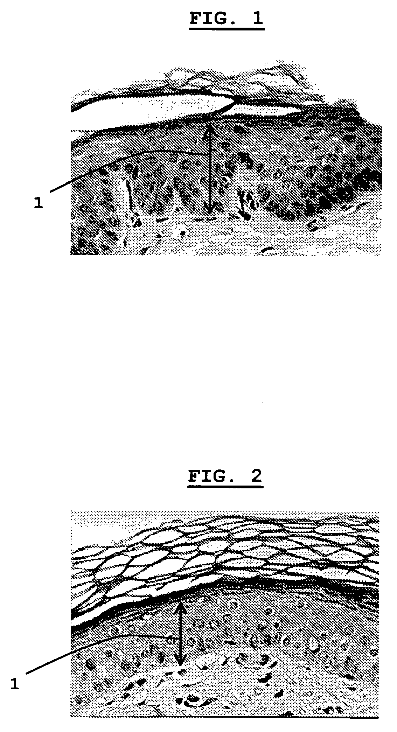

La figure 2 montre une disparition des zones sombres au niveau de l'épiderme 1 par rapport à la figure 1. Autrement dit, on observe sur les zones traitées une diminution importante de la mélanine dans l'épiderme 1, diminution que l'on peut donc corréler à l'éclaircissement de la peau observé à l'oeil nu.

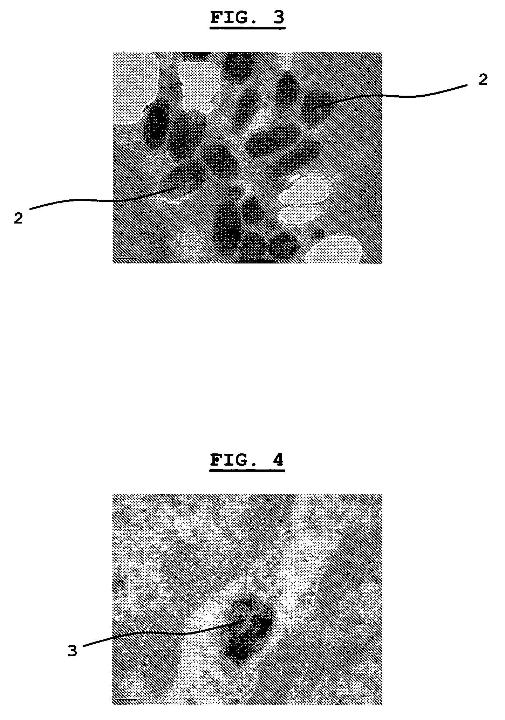

La figure 3 montre un échantillon non traité dans lequel les mélanosomes 2 ont une structure homogène, alors qu'après traitement, la figure 4 fait apparaître un mélanosome 3 présentant une structure hétérogène illustrant un processus de digestion de la mélanine en cours.

Ces études ont confirmé la disparition de la mélanine épidermique coexistant avec une synthèse normale de mélanine par les mélanocytes dans l'épiderme au niveau des zones traitées. Ceci tend à prouver que l'apport local de phosphatases acides a favorisé la dégradation de mélanine.

Chez l'animal, ont donc été obtenues une bonne efficacité d'éclaircissement et une bonne tolérance de la composition selon l'invention.

### Exemple 1B : Etudes chez l'homme.

Une étude partielle a été réalisée sur des sujets porteurs de mélasma et n'ayant pas répondu aux traitements existant actuellement sur le marché.

Une solution de phosphatases acides identique à celle de l'exemple 1A a été appliquée sur les zones du visage à traiter une fois par jour.

Il est ressorti de cette étude que la tolérance de la solution de phosphatases acides était parfaite et que les effets éclaircissants étaient bons. Par exemple, on a observé une atténuation de l'hyperpigmentation du mélasma au bout de trois semaines dans 70% des cas.

### Exemple 2 : Utilisation d'un mélange de phosphatases acides et d'un agent dépigmentant pour éclaircir la peau.

Une solution contenant un mélange de phosphatases acides et de N-acétylcystéine en tant qu'agent dépigmentant a été utilisée pour les études chez l'animal et chez l'homme présentées dans les exemples 2A et 2B.

La solution contenait 5% en poids de phosphatases acides d'origine végétale et 5% en poids de N-acétylcystéine, dans de l'eau et du propylène glycol.

### Exemple 2A : Etudes chez l'animal.

Le même protocole que dans l'exemple 1A a été suivi avec la solution contenant le mélange phosphatases acides et N-acétylcystéine à la place de la solution de phosphatases acides sans agent dépigmentant additionnel.

Chez l'animal, l'efficacité d'éclaircissement d'un mélange de phosphatases acides extraites de végétaux et de N-acétylcystéine est supérieure à celle de la solution isolée de phosphatases acides.

La tolérance est au contraire moins bonne en raison de la présence d'un dérivé de la cystéine, souvent irritant pour la peau.

### Exemple 2B : Etudes chez l'homme.

Le même protocole que dans l'exemple 1B a été suivi avec la solution contenant le mélange phosphatases acides et N-acétylcystéine à la place de la solution de phosphatases acides sans agent dépigmentant additionnel, chez des sujets porteurs de mélasmas rebelles.

Les résultats observés chez l'animal (exemple 2A) ont ainsi été confirmés chez l'homme.

Avec une telle composition, qui à la fois favorise la dégradation de la mélanine et à la fois freine sa synthèse, on gagne en efficacité d'éclaircissement, mais la tolérance est moins bonne que celle observée avec une composition contenant uniquement des phosphatases acides sans agent dépigmentant additionnel.

## Revendications

1. Utilisation cosmétique d'au moins une phosphatase acide pour éclaircir la peau.

2. Utilisation selon la revendication 1, dans laquelle les phosphatases acides sont d'origine végétale.

3. Utilisation selon la revendication 1 ou 2, dans laquelle ladite au moins une phosphatase acide est extraite de la pomme de terre, de la patate douce, ou d'une céréale telle que le germe de blé.

## Patentansprüche

1. Kosmetische Verwendung mindestens einer sauren Phosphatase zur Aufhellung der Haut.

2. Verwendung nach Anspruch 1, wobei die sauren Phosphatasen pflanzlichen Ursprungs sind.

3. Verwendung nach Anspruch 1 oder 2, wobei mindestens eine saure Phosphatase aus Kartoffeln, Süsskartoffeln oder einem Getreide wie Weizenkeimen gewonnen wird.

## Claims

1. Cosmetic use of at least one acid phosphatase to lighten the skin.

2. The use according to claim 1, wherein the acid phosphatases are of plant origin.

3. The use according to claim 1 or 2, wherein said at least one acid phosphatase is extracted from potato, sweet potato, or cereal such as wheat germ.
